# EUROPEAN PATENT APPLICATION

(11) **EP 3 838 162 A1**
(43) Date of publication of application: **23.06.2021**
(21) Application number: 19290117.1
(22) Date of filing: 16.12.2019
(51) Int. Cl.: A61B 8/08

(54) **SYSTEMS AND METHODS FOR ASSESSING A PLACENTA**

(71) Applicant: Koninklijke Philips N.V., 5656 AG Eindhoven (NL)
(72) Inventor: Lefevre, Thierry, 5656 AE Eindhoven (NL); Raynaud, Caroline Denise Francoise, 5656 AE Eindhoven (NL); Rouet, Laurence, 5656 AE Eindhoven (NL); Ciofolo-Veit, Cybèle, 5656 AE Eindhoven (NL)
(74) Representative: de Haan, Poul Erik

(57) **Abstract**

The invention provides a method for performing an assessment of a placenta. The method includes obtaining a 3D ultrasound image of a uterus (210) and segmenting the placenta (220). A 3D rendering (200) of the uterus is then generated, wherein the generating includes: identifying a position of the placenta within the uterus (such as the position of the placenta relative to the cervix 250) ; obtaining anatomical reference data relating to a potential risk associated with the position of the placenta within the uterus; and comparing the position of the placenta and the anatomical reference data. A 3D rendering of the uterus is generated that comprises a 3D rendering of the placenta, marked with an indicator that is altered based on the comparison of the position of the placenta and the anatomical reference data. The appearance of the indicator may vary according to e.g. risk type/severity.

## Description

### FIELD OF THE INVENTION

This invention relates to the field of uterine imaging, and more specifically to the field of placental assessment based on a uterine image.

### BACKGROUND OF THE INVENTION

As the organ that provides a fetus with blood, oxygen and nutrients, the health of the placenta is of critical importance during pregnancy. Placental abnormalities can be benign or can require the implementation of a specific pregnancy management technique. For example, the position of the placental implantation may be highly variable within the uterus. Further, scars resulting from the increasing number of C-section deliveries are a frequent source of placental anomalies in subsequent pregnancies.

In some cases, the placenta assessment may reveal a severe medical condition, such as a morbidly adherent placenta, which requires a specific delivery plan to ensure the survival of both mother and child.

Typically, it is difficult to observe the placenta in its totality because of its large size, particularly after the first trimester of pregnancy. Further, specific skills are also needed by a sonographer to find and assess placental abnormalities accurately.

There is therefore a need for a means to accurately assess a placenta within a uterine image.

### SUMMARY OF THE INVENTION

The invention is defined by the claims.

According to examples in accordance with an aspect of the invention, there is provided a method for performing an assessment of a placenta, the method comprising:
obtaining a 3D ultrasound image of a uterus, the uterus containing a placenta;
segmenting the placenta from the 3D ultrasound image of the uterus by way of an image segmentation algorithm; and
generating a 3D rendering of the uterus based on the result of the segmentation of the placenta and the 3D ultrasound image of the uterus, wherein generating the 3D rendering of the uterus comprises:
   identifying a position of the placenta within the uterus;
   obtaining anatomical reference data relating to a potential risk associated with the position of the placenta within the uterus;
   comparing the position of the placenta and the anatomical reference data;
   generating a 3D rendering of the uterus based on the 3D ultrasound image of the uterus, the 3D rendering of the uterus comprising a 3D rendering of the placenta; and
   marking the 3D rendering of the placenta with an indicator, wherein the indicator is altered based on the comparison of the position of the placenta and the anatomical reference data.

The method provides a means of assessing an entire uterus and placenta, which are typically too large to fit within a single ultrasound image, particularly after the first trimester of pregnancy.

By generating a full 3D rendering of the uterus based on the image data and the result of the segmentation of the placenta, it is possible to present the user with a visual representation of the scanned area with an automatically determined indicator to emphasize an aspect of the placenta based on its position within the uterus.

In this way, the assessment of a placenta, and determining possible considerations based on the position of the placenta within the uterus, may be simplified.

In an embodiment, the method comprises:
obtaining a plurality of 3D ultrasound images; and
combining the plurality of 3D ultrasound images to form the 3D ultrasound image of the uterus.

In this way, the method provides a means of assessing a uterus when it is too large to fit within a single field of view.

In an embodiment, the method further comprises segmenting an additional anatomical structure within the 3D ultrasound image of the uterus, and wherein generating the 3D rendering of the uterus further comprises:
identifying a position of the placenta with respect to the additional anatomical structure;
obtaining additional anatomical reference data relating to a potential risk associated with the position of the placenta with respect to the additional anatomical structure;
comparing the position of the placenta and the additional anatomical reference data;
marking one or more of the 3D rendering of the placenta and the 3D rendering of the uterus with an additional indicator, wherein the additional indicator is altered based on the comparison of the position of the placenta and the additional anatomical reference data.

In this way, the interaction of the placenta and another physical structure in the vicinity of the uterus may be determined and highlighted to a user in the 3D rendering of the uterus.

In a further embodiment, the additional anatomical structure comprises one or more of:
a bladder;
an umbilical cord;
a cervix;
a cervical area; and
an anatomical structure in the vicinity of the uterus.

In an embodiment, the method further comprises determining a physical characteristic of the placenta based on the result of the segmentation of the placenta.

In this way, it is possible to perform additional assessments of the placenta and the uterus based on various physical aspects of the placenta.

In a further embodiment, the physical characteristic comprises one or more of:
a placenta volume; and
a placenta texture

In an embodiment, the method further comprises detecting a placental abnormality, such as a placental lake, within the placenta, and wherein generating the 3D rendering of the uterus further comprises marking the placental lake with a lake indicator.

In this way, an additional measure of placenta health may be automatically obtained from the image data and highlighted to the user in the 3D rendering of the uterus.

In an embodiment, the method further comprises detecting a placenta implantation type based on the result of the segmentation of the placenta and the 3D ultrasound image of the uterus, and wherein generating the 3D rendering of the uterus further comprises marking the 3D rendering of the placenta with an implantation type indicator.

In this way, an additional measure of placenta health may be automatically obtained from the image data and highlighted to the user in the 3D rendering of the uterus.

In a further embodiment, detecting the placenta implantation type comprises:
determining a thickness of a wall of the uterus in the vicinity of the placenta; and
identifying the placenta implantation type based on the thickness of the wall of the uterus.

In an embodiment, detecting the placenta implantation type is performed using a machine learning algorithm.

In an embodiment, the method further comprises performing a Doppler ultrasound based vascular assessment of the placenta.

In this way, an additional measure of placenta health may be automatically obtained from the image data and highlighted to the user in the 3D rendering of the uterus.

According to examples in accordance with an aspect of the invention, there is provided a computer program comprising computer program code means which is adapted, when said computer program is run on a computer, to implement the methods described above.

According to examples in accordance with an aspect of the invention, there is provided an ultrasound system for assessing a placenta, the ultrasound system comprising:
an ultrasound probe adapted to acquire a 3D ultrasound image of a uterus and a placenta;
a processor adapted to:
   segment the placenta from the 3D ultrasound image of the uterus by way of an image segmentation algorithm; and
   generate a 3D rendering of the uterus based on the result of the segmentation of the placenta and the 3D ultrasound image of the uterus, wherein generating the 3D rendering of the uterus comprises:
      identify a position of the placenta within the uterus;
      obtain anatomical reference data relating to a potential risk associated with the position of the placenta within the uterus;
      compare the position of the placenta and the anatomical reference data;
      generate a 3D rendering of the uterus based on the 3D ultrasound image of the uterus, the 3D rendering of the uterus comprising a 3D rendering of the placenta; and
      mark the 3D rendering of the placenta with an indicator, wherein the indicator is altered based on the comparison of the position of the placenta and the anatomical reference data; and
   a display unit adapted to display the 3D rendering of the uterus to a user.

In an embodiment, the ultrasound system further comprises a user interface adapted to receive a user input indicating a region of interest for further investigation on the 3D rendering of the uterus, and wherein the processor is adapted to:
determine a set of acquisition settings based on the indicated region of interest; and
apply the set of acquisition settings to the ultrasound system.

In this way, the user may simply indicate an area requiring further investigation in order to prepare the system for performing an additional acquisition using the optimal settings.

In an embodiment, a further 3D ultrasound image of the uterus is acquired, and wherein the processor is further adapted to update the 3D rendering of the uterus based on the further 3D ultrasound image of the uterus.

In this way, any further investigations performed by the user may be automatically added to the 3D rendering of the uterus.

These and other aspects of the invention will be apparent from and elucidated with reference to the embodiment(s) described hereinafter.

### BRIEF DESCRIPTION OF THE DRAWINGS

Examples of the invention will now be described in detail with reference to the accompanying drawings, in which:
Figure 1 shows an ultrasound diagnostic imaging system to explain the general operation;
Figure 2 shows a method of the invention; and
Figure 3 shows an example of a 3D rendering of a uterus.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for performing an assessment of a placenta. The method includes obtaining a 3D ultrasound image of a uterus, which contains a placenta, and segmenting the placenta from the 3D ultrasound image of the uterus by way of an image segmentation algorithm.

A 3D rendering of the uterus is then generated based on the result of the segmentation of the placenta and the 3D ultrasound image of the uterus. Generating the 3D rendering of the uterus includes: identifying a position of the placenta within the uterus; obtaining anatomical reference data relating to a potential risk associated with the position of the placenta within the uterus; and comparing the position of the placenta and the anatomical reference data. A 3D rendering of the uterus is generated based on the 3D ultrasound image of the uterus, the 3D rendering of the uterus comprising a 3D rendering of the placenta and the 3D rendering of the placenta is marked with an indicator, wherein the indicator is altered based on the comparison of the position of the placenta and the anatomical reference data.

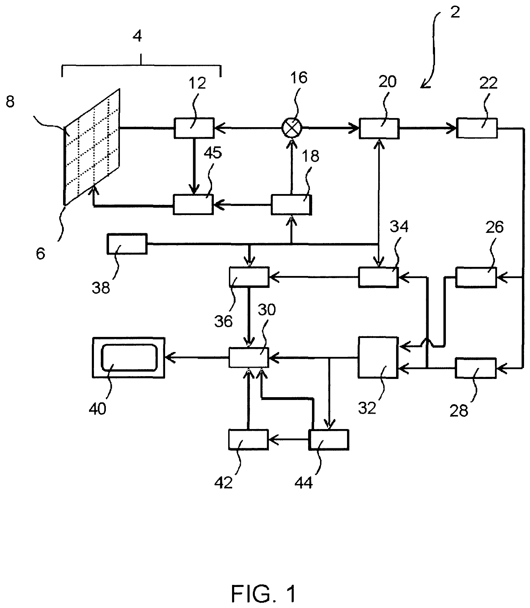

The general operation of an exemplary ultrasound system will first be described, with reference to Figure 1.

The system comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Figure 1 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

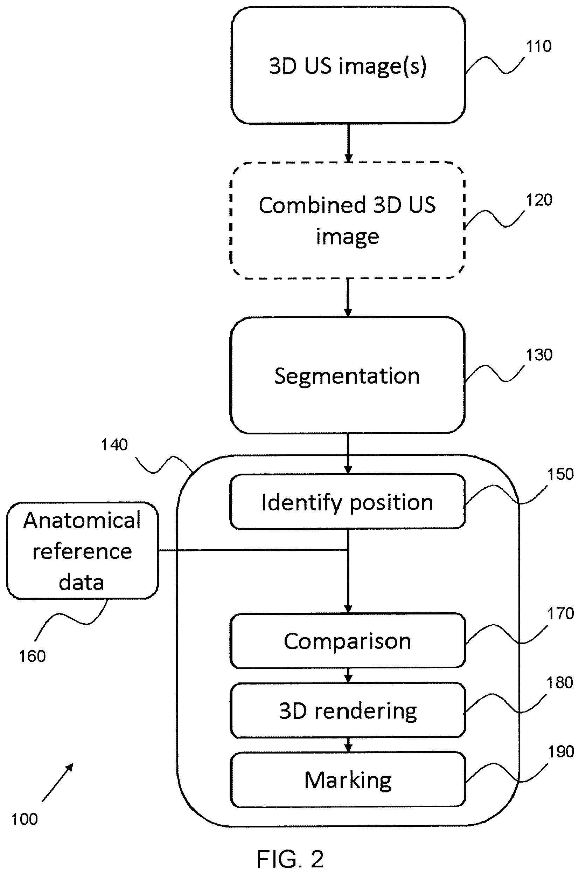

Figure 2 shows a method 100 for performing an assessment of a placenta according to an aspect of the invention.

The method begins in step 110 by obtaining a 3D ultrasound image of a uterus, the uterus containing a placenta.

In the early stages of pregnancy, the acquisition of the entire uterus and placenta is possible with a single sweep of a 3D ultrasound probe. Later, when the pregnancy has progressed to a more advanced stage, the uterus will likely not fit in a single ultrasound acquisition volume; however, it is possible to cover the whole area of the uterus with a series of multiple volume acquisitions.

In other words, a plurality of 3D ultrasound images may be obtained in order to assess a larger area, in which case the method may include step 120, wherein the plurality of 3D ultrasound images are combined to form the 3D ultrasound image of the uterus.

Using the plurality of 3D ultrasound images, and in some cases in combination with spatial ultrasound probe position tracking obtained, for example, by way of electromagnetic tracking, a 3D image of the whole uterus may be reconstructed using a volume fusion algorithm. Such a reconstruction may be possible even if the fetus is moving during the acquisition of the plurality of 3D ultrasound images because the uterus, and the placenta, will remain stable.

In step 130, the placenta is segmented from the 3D ultrasound image of the uterus by way of an image segmentation algorithm. Any suitable image segmentation algorithm may be used.

In the context of the present invention, segmenting an object or a structure preferably refers to finding the contours or borders of said object or structure in a 3D volume. Accordingly, the expression "segmenting the placenta" preferably refers to finding the contours or borders of the placenta in a representation of the same, such as *inter alia* an image, a mask (e.g., a binary mask) or a mesh.

For example, once the plurality of 3D ultrasound images of the uterus have been combined, the placenta is segmented from the 3D ultrasound image of the uterus. The placenta segmentation may be performed, for example, using a 3D deep-learning segmentation algorithm such as a U-net architecture as described O. Ronneberger et al. U-Net: Convolutional Networks for Biomedical Image Segmentation, In Proceedings of MICCAI'15. Vol 9351, 2015, p. 234-241.

In step 140, a 3D rendering of the uterus is generated based on the result of the segmentation of the placenta and the 3D ultrasound image of the uterus. Generating the 3D rendering of the uterus includes the following steps.

In step 150, the position of the placenta within the uterus is identified. The position of the placenta within the uterus may be identified based on the result of the segmentation of the placenta.

In addition, the position of the placenta relative to the cervix, which may be detected within the 3D ultrasound image of the uterus (for example, by way of a shape recognition algorithm or a machine learning algorithm) is identified. The position of the cervix within the uterus and the position of the placenta relative to the cervix may then be compared to the anatomical reference data in the following step.

In step 160, anatomical reference data relating to a potential risk associated with the position of the placenta within the uterus is obtained. The anatomical reference data may be obtained from a local memory of the ultrasound system, or from a remote memory, for example held on a server.

In step 170, the position of the placenta is compared the anatomical reference data in order to determine a state of the placenta, and uterus, based on the reference data.

In step 180, a 3D rendering of the uterus is generated based on the 3D ultrasound image of the uterus, the 3D rendering of the uterus comprising a 3D rendering of the placenta and the 3D rendering of the placenta is marked in step 190 with an indicator, wherein the indicator is altered based on the comparison of the position of the placenta and the anatomical reference data.

Put another way, a global 3D visualization of the placenta in the uterus is generated, using a 3D rendering engine, on which the areas of interest for the clinician that have been segmented in the preceding steps are marked with an indicator, for example by being highlighted with colors or a specific light effect. The indicator may include, for example, colored overlays over areas of interest of the 3D rendering of the uterus or synthetic structure rendering, such as a mesh on the 3D rendering of the uterus based on the results of the segmentation.

The indicator may be any type of indicator suitable for communicating information to a user. For example, the indicator may make use of a variety of colors, with an associated key, in order to convey information to the user. Alternatively, or in addition to the variety of colors, the indicator may have a variable brightness, saturation or pattern in order to convey the desired information.

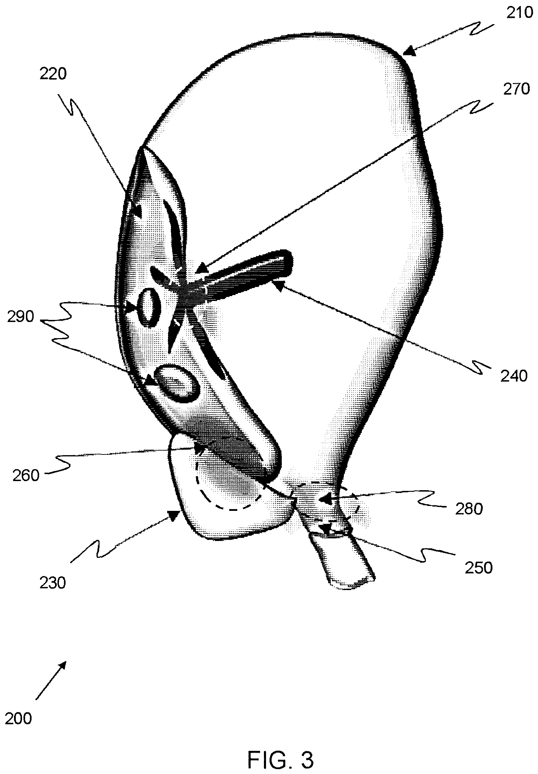

Figure 3 shows an example of a 3D rendering 200 of a uterus 210.

In the example shown in Figure 3, the 3D rendering has been generated in a method similar to the method described above with respect to Figure 2, wherein the method further includes segmenting one or more additional anatomical structures within the 3D ultrasound image of the uterus. In this case, generating the 3D rendering of the uterus further comprises identifying the position of the placenta 220 with respect to the one or more additional anatomical structures. Once again, anatomical reference data relating to a potential risk associated with the position of the placenta with respect to the additional anatomical structure and the position of the placenta is compared to the anatomical reference data. The 3D rendering of the placenta and/or the 3D rendering of the uterus may then be marked with an additional indicator, wherein the additional indicator is altered based on the comparison of the position of the placenta and the additional anatomical reference data.

Looking to Figure 3, the additional anatomical structures include: a bladder 230; an umbilical cord 240; and a cervix 250. In other words, the bladder, the umbilical cord and the cervix are segmented from the 3D ultrasound image of the uterus and rendered as part of the 3D rendering 200.

Using the obtained anatomical reference data, the locations of the additional anatomical structures, within the uterus 210 and with respect to the placenta 220, may be assessed to assess a potential health risk or condition associated with the uterine layout. In response to this assessment, the areas of importance may be marked in a similar manner to the marking of the 3D rendering of the placenta as described above.

In the example shown in Figure 3, the 3D rendering 200 of the uterus 210 includes three areas of interest based on the additional anatomical structures segmented from the 3D ultrasound image of the uterus. In particular, there is present a bladder/uterus interface 260, an umbilical cord insertion area 270 and a cervical area 280. Each of the areas of interest may be marked with an individual indicator, wherein the appearance of the indicator varies according to, for example, area type or risk type/severity.

The additional anatomical structures may be segmented using similar methods to the methods described above with relation to the placenta segmentation.

Examples of risk assessment for each additional anatomical structure may include, for the cervical area 280, if the placenta 210 is close to, or covering, the cervix 250 (referred to as placenta praevia), a specific pregnancy management, possibly including a C-section delivery, may be identified as being required. In the case of the cord insertion area, if the umbilical cord is inserted close to the edge of the placenta, specific vascular conditions may occur and require attention during or following the pregnancy.

In the case of the bladder/uterus interface, if the placenta is covering this area, a specific Doppler examination can be performed to assess the vascularity around the bladder. An increased vascularity may be an indicator of possible myometrial invasion and a morbidly adherent placenta, which requires specific pregnancy management and delivery planning.

Further, the 3D rendering of the uterus may be used for the purposes of abnormality detection and quantification.

For example, the 3D rendering of the uterus, and in particular the 3D rendering of the placenta may be used to assess a placental implantation, which relates to the region of the uterus in which the placenta is located.

Put another way, the methods described above may further comprise detecting a placenta implantation type based on the result of the segmentation of the placenta and the 3D ultrasound image of the uterus, and wherein generating the 3D rendering of the uterus further comprises marking the 3D rendering of the placenta with an implantation type indicator.

In other words, the 3D visualization may display suspicious placental implantation in the uterine wall.

For example, the thickness and appearance (texture) of the myometrium (uterine wall) in the placental implantation region may indicate a possible abnormal placental implantation. The texture of the myometrium may be classified as normal or abnormal with an image classification deep-learning algorithm.

Further, the placenta 220 may comprise one or more placental lakes 290, which are enlarged spaces within the placenta filled with blood.

Accordingly, the methods described above may further comprise detecting a placental lake within the placenta and marking the placental lake in the 3D rendering of the uterus with a lake indicator.

The detection of placental lakes may be combined with determining additional physical characteristics of the placenta other than the placental position within the uterus, such as the volume of the placenta, which may be derived from the segmentation of the placenta. In a similar manner, the volume of the placental lakes may be determined, such as by way of texture analysis and region-based segmentation, and so a proportion of the placenta occupied by the placental lakes may be determined.

It will be appreciated that the image processing methods described above may make use of a variety of machine learning algorithms, in particular for the purposes of feature recognition and image segmentation.

The system described above with respect to Figure 1, or any other system capable of performing the methods described above, may include a user interface adapted to receive a user input indicating a region of interest for further investigation on the 3D rendering of the uterus. For example, the 3D rendering may be displayed on a touchscreen display, which the user may interact with by touching a given area of interest for further investigation.

In an example, in order to improve quantitative assessment of the uterus, each area of the 3D rendered visualization may be indicated, or manually selected by a user, to display the corresponding quantification and suggest the clinician to do additional acquisitions (vascular Doppler for example).

Further, the 3D visualization may be used to initiate additional acquisitions, such as vascular Doppler assessment in the placental lakes or at the border with the uterine wall. For example, the user may manually select an area of interest, which may launch an additional acquisition, for example a vascular Doppler acquisition.

Put another way, when the user indicates a region of interest on the 3D rendering of the uterus, the system may propose to perform a vascular Doppler acquisition to show, for example, specific flow patterns in placental lakes or increased vascularity, especially at the interface of the uterus with the bladder.

The spatial coordinates of the area of interest may be stored and the ultrasound system may be automatically prepared for the selected additional acquisition with the optimal settings based on the area of the additional acquisition.

Once the additional acquisition has been performed, the results, such as a flow visualization from a vascular Doppler acquisition, are displayed both on the usual screen and on the 3D rendered volume. This may be done by using the area of interest coordinates that were previously stored to register the additional acquisition result and update the global 3D rendering.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A method (100) for performing an assessment of a placenta, the method comprising:
obtaining (110) a 3D ultrasound image of a uterus, the uterus containing a placenta;
segmenting (130) the placenta from the 3D ultrasound image of the uterus by way of an image segmentation algorithm; and
generating (140) a 3D rendering of the uterus based on the result of the segmentation of the placenta and the 3D ultrasound image of the uterus, wherein generating the 3D rendering of the uterus comprises:
identifying (150) a position of the placenta within the uterus;
obtaining (160) anatomical reference data relating to a potential risk associated with the position of the placenta within the uterus;
comparing (170) the position of the placenta and the anatomical reference data;
generating (180) a 3D rendering of the uterus based on the 3D ultrasound image of the uterus, the 3D rendering of the uterus comprising a 3D rendering of the placenta; and
marking (190) the 3D rendering of the placenta with an indicator, wherein the indicator is altered based on the comparison of the position of the placenta and the anatomical reference data.

2. A method as claimed in claim 1, wherein the method comprises:
obtaining a plurality of 3D ultrasound images; and
combining (120) the plurality of 3D ultrasound images to form the 3D ultrasound image of the uterus.

3. A method (100) as claimed in any of claims 1 to 2, wherein the method further comprises segmenting an additional anatomical structure within the 3D ultrasound image of the uterus, and wherein generating the 3D rendering of the uterus further comprises:
identifying a position of the placenta with respect to the additional anatomical structure;
obtaining additional anatomical reference data relating to a potential risk associated with the position of the placenta with respect to the additional anatomical structure;
comparing the position of the placenta and the additional anatomical reference data;
marking one or more of the 3D rendering of the placenta and the 3D rendering of the uterus with an additional indicator, wherein the additional indicator is altered based on the comparison of the position of the placenta and the additional anatomical reference data.

4. A method (100) as claimed in claim 3, wherein the additional anatomic structure comprises one or more of:
a bladder;
an umbilical cord;
a cervix;
a cervical area; and
an anatomical structure in the vicinity of the uterus.

5. A method (100) as claimed in any of claims 1 to 4, wherein the method further comprises determining a physical characteristic of the placenta based on the segmentation of the placenta.

6. A method (100) as claimed in claim 5, wherein the physical characteristic comprises one or more of:
a placenta volume; and
a placenta texture.

7. A method (100) as claimed in any of claims 1 to 6, wherein the method further comprises detecting a placental abnormality, such as a placental lake, within the placenta, and wherein generating the 3D rendering of the uterus further comprises marking the placental lake with a lake indicator.

8. A method (100) as claimed in any of claims 1 to 7, wherein the method further comprises detecting a placenta implantation type based on the result of the segmentation of the placenta and the 3D ultrasound image of the uterus, and wherein generating the 3D rendering of the uterus further comprises marking the 3D rendering of the placenta with an implantation type indicator.

9. A method (100) as claimed in claim 8, wherein detecting the placenta implantation type comprises:
determining a thickness of a wall of the uterus in the vicinity of the placenta; and
identifying the placenta implantation type based on the thickness of the wall of the uterus.

10. A method (100) as claimed in any of claims 8 to 9, wherein detecting the placenta implantation type is performed using a machine learning algorithm.

11. A method (100) as claimed in any of claims 1 to 10, wherein the method further comprises performing a Doppler ultrasound based vascular assessment of the placenta.

12. A computer program comprising computer program code means which is adapted, when said computer program is run on a computer, to implement the method of any of claims 1 to 11.

13. An ultrasound system (2) for assessing a placenta, the ultrasound system comprising:
an ultrasound probe (4) adapted to acquire a 3D ultrasound image of a uterus and a placenta;
a processor adapted to:
segment the placenta from the 3D ultrasound image of the uterus by way of an image segmentation algorithm; and
generate a 3D rendering of the uterus based on the result of the segmentation of the placenta and the 3D ultrasound image of the uterus, wherein generating the 3D rendering of the uterus comprises:
identify a position of the placenta within the uterus;
obtain anatomical reference data relating to a potential risk associated with the position of the placenta within the uterus;
compare the position of the placenta and the anatomical reference data;
generate a 3D rendering of the uterus based on the 3D ultrasound image of the uterus, the 3D rendering of the uterus comprising a 3D rendering of the placenta; and
mark the 3D rendering of the placenta with an indicator, wherein the indicator is altered based on the comparison of the position of the placenta and the anatomical reference data; and
a display unit (40) adapted to display the 3D rendering of the uterus to a user.

14. A, ultrasound system as claimed in claim 13, wherein the ultrasound system further comprises a user interface adapted to receive a user input indicating a region of interest for further investigation on the 3D rendering of the uterus, and wherein the processor is adapted to:
determine a set of acquisition settings based on the indicated region of interest; and
apply the set of acquisition settings to the ultrasound system.

15. An ultrasound system as claimed in claim 14, wherein a further 3D ultrasound image of the uterus is acquired, and wherein the processor is further adapted to update the 3D rendering of the uterus based on the further 3D ultrasound image of the uterus.
